(19) Europäisches Patentamt
European Patent Office
Office européen des brevets

(11) **EP 4 434 512 A1**

## EUROPEAN PATENT APPLICATION
published in accordance with Art. 153(4) EPC

(43) Date of publication:
**25.09.2024 Bulletin 2024/39**

(21) Application number: **22892700.0**

(22) Date of filing: **04.11.2022**

(51) International Patent Classification (IPC):
***A61K 6/887*** (2020.01)        ***A61K 6/842*** (2020.01)
***A61K 6/878*** (2020.01)

(52) Cooperative Patent Classification (CPC):
**A61K 6/842; A61K 6/878; A61K 6/887**

(86) International application number:
**PCT/JP2022/041134**

(87) International publication number:
**WO 2023/085201 (19.05.2023 Gazette 2023/20)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **15.11.2021   JP 2021185799**

(71) Applicant: **Tokuyama Dental Corporation
Tokyo 110-0016 (JP)**

(72) Inventors:
• **MIRISAKI, Hiroshi
Tokyo 110-0016 (JP)**
• **KIRA, Ryuta
Tokyo 110-0016 (JP)**
• **AKIZUMI, Hironobu
Tokyo 110-0016 (JP)**

(74) Representative: **Hoffmann Eitle
Patent- und Rechtsanwälte PartmbB
Arabellastraße 30
81925 München (DE)**

(54) **DENTAL CURABLE COMPOSITION**

(57)     The dental curable composition of the present invention contains 100 parts by mass of a polymerizable monomer, 170 to 270 parts by mass of an inorganic filler (B) having an average particle diameter of 50 nm to 1 $\mu$m and having a zeta potential of a constituent particle with either positive or negative polarity, and 5 to 50 parts by mass of an inorganic filler (C) composed of inorganic particles having a zeta potential opposite to that of (B) and having a specific surface area of 25 to 100 $m^2$/g. According to the present invention, it is possible to pro-vide a dental curable composition containing a polymerizable monomer and an inorganic filler, which can be suitably used as a flowable composite resin, the dental curable composition having a consistency suitable for discharge from a syringe equipped with a needle tip and good shapability without treating the inorganic filler with a special surface treating agent and without being affected by the type of the polymerizable monomer used, and being particularly suitably used as a flowable composite resin.

EP 4 434 512 A1

**Description**

Technical Field

The present invention relates to a dental curable composition having good operability that can be suitably used as a dental material, particularly a dental flowable composite resin, which can substitute for a part or the whole of a natural tooth in the field of dental medicine.

Background Art

A dental curable composition contains, as main components, a polymerizable monomer (monomer), an inorganic filler (filler) composed of inorganic particles (inorganic powder), and a polymerization initiator, and among them, a composite resin is one of the materials most frequently used in dental treatment as a material for repairing a tooth defect or a cavity after removing dental caries.

A composite resin is required to be excellent in various physical properties such as operability in a paste state before polymerization and curing, and aesthetics and mechanical strength of a cured product obtained after polymerization and curing.

In recent years, a flowable composite resin has been developed which is designed to be able to directly fill a cavity with paste from the tip of a needle called a needle tip, by mounting a needle having a small hole on a syringe containing a composite resin paste, and is used more frequently in clinical sites because it allows for easier restoration of teeth.

The flowable composite resin is required to have good operability in filling the flowable composite resin into a cavity or the like, specifically, good operability in discharging the flowable composite resin from a syringe equipped with a needle tip, and good shapability so that the flowable composite resin is not deformed during the period from shaping after filling to curing (property that is less likely to be deformed by natural flow during standing and can maintain its shape).

However, when the above operability is enhanced by increasing the fluidity of the paste by reducing the content of the inorganic filler as compared with a general composite resin (universal type), the so-called "sag property" is also increased with the improvement in fluidity, and thus the shapability tends to be decreased. As the technique to solve this problem, a method in which an inorganic filler is surface-treated with a silane coupling agent having a long-chain alkylene group to highly increase the affinity between the inorganic filler and a polymerizable monomer, thereby appropriately maintaining the consistency without reducing the shapability, is known. Here, the "consistency" is an index representing the hardness (softness) of the fluid paste, and the above-mentioned "appropriate consistency" means such a paste hardness that the discharge amount can be arbitrarily controlled by slight force adjustment without largely increasing the force applied to the piston when the fluid paste is discharged from the syringe equipped with the needle tip.

For example, PTL 1 discloses "a dental curable composition, which contains a polymerizable monomer (A), irregularly shaped inorganic particles (B) having an average particle diameter of 0.1 to 0.3 $\mu$m which are surface-treated with a silane coupling agent (a) having an alkylene group with a specific chain length, and inorganic ultrafine particles (C) having an average particle diameter of 5 to 50 nm which are surface-treated with a silane coupling agent (b) having an alkylene group with a chain length different from that of the silane coupling agent (a), and in which a content of (B) in a total of the (B) and the (C) is 92.5 to 98% by weight".

In addition, PTL 2 discloses "a dental curable composition, which contains a polymerizable monomer, a filler (A) composed of inorganic particles surface-treated with a silane coupling agent (1) having an alkylene group with a specific chain length, and a filler (B) composed of inorganic particles surface-treated with a silane coupling agent having an alkylene group with a chain length different from that of the silane coupling agent (1), in which an average particle diameter of the filler (B) is 20% or more and 550% or less with respect to an average particle diameter of the filler (A)".

Citation List

Patent Literature

PTL1: WO 2014/083842 A
PTL2: WO 2020/031444 A

Summary of Invention

Technical Problem

The dental curable compositions described in PTLs 1 and 2 are said to have appropriate consistency and good shapability required for a flowable composite resin. However, since both of PTLs 1 and 2 require the use of inorganic particles surface-treated with a silane coupling agent having a long-chain alkylene group, there is a concern that not only the production method is limited but also the polymerizable monomer is limited in terms of composition. That is, since the silane coupling agent having a long-chain alkylene group generally has high hydrophobicity and is water-insoluble, it is necessary to remove an organic solvent after performing the treatment in a state of being dissolved in a water-insoluble organic solvent such as toluene, and since the reactivity with respect to the inorganic particles is also low, it is necessary to pay attention to the surface treatment. Furthermore, when a polymerizable monomer containing a large amount (for example, a proportion of about 20 to 45% by mass in the total polymerizable monomer)of a highly hydrophilic compound such as triethylene glycol dimethacrylate (3G) having a low affinity for the silane coupling agent is used, there is a concern that the desired effect cannot be obtained.
Therefore, an object of the present invention is to provide a dental curable composition which can be suitably used as a flowable composite resin, which has an appropriate consistency when discharged from a syringe equipped with a needle tip without subjecting inorganic particles to a surface treatment using a silane coupling agent having a long-chain alkylene group (as disclosed in PTL 1 and PTL 2), which has good shapability after discharge, and which can exhibit such properties even when a hydrophilic polymerizable monomer is contained in a large amount.

Solution to Problem

The present invention solves the above problem, and a first aspect of the present invention is a dental curable composition containing: a polymerizable monomer (A); an inorganic filler (B) having an average particle diameter of 50 nm to 1 $\mu$m, in which a polarity of a zeta potential of the inorganic filler measured in water is either negative (minus) or positive (plus), and the polarity is constant between inorganic particles constituting the inorganic filler; and an inorganic filler (C) having a polarity of a zeta potential measured in water that is opposite to the polarity of the inorganic filler (B) and a specific surface area measured by a nitrogen adsorption method of 25 to 100 $m^2/g$; in which blending amounts of the inorganic filler (B) and the inorganic filler (C) with respect to 100 parts by mass of the polymerizable monomer (A) are 170 to 270 parts by mass of the inorganic filler (B) and 5 to 50 parts by mass of the inorganic filler (C), respectively.
In the dental curable composition of the above-described aspect (hereinafter, also referred to as "dental curable composition of the present invention"), it is preferable that a polarity of a zeta potential of the inorganic filler (B) measured in water is negative (minus). The proportion of the mass of the inorganic filler (C) to the total mass of the inorganic filler (B) and the inorganic filler (C) is preferably 1 to 20% by mass. Furthermore, it is preferable that the polymerizable monomer (A) is composed of a (meth)acrylic polymerizable monomer, the inorganic filler (B) is composed of silica-based composite oxide particles, and the inorganic filler (C) is composed of crystalline rare earth metal fluoride particles, in which the particles have a full width at half maximum of a maximum intensity peak derived from the crystalline rare earth metal fluoride in an X-ray diffraction pattern of the particles of 0.3° or more.
A second aspect of the present invention is a flowable composite resin including the dental curable composition of the present invention.
In the present invention, the following [1] to [10] can be provided, including the above-mentioned first and second aspects.

[1] A dental curable composition containing: a polymerizable monomer (A); an inorganic filler (B) having an average particle diameter of 50 nm to 1 $\mu$m, in which a polarity of a zeta potential of the inorganic filler measured in water is either negative (minus) or positive (plus), and the polarity is constant between inorganic particles constituting the inorganic filler; and an inorganic filler (C) having a polarity of a zeta potential measured in water that is opposite to the polarity of the inorganic filler (B) and a specific surface area measured by a nitrogen adsorption method of 25 to 100 $m^2/g$, in which blending amounts of the inorganic filler (B) and the inorganic filler (C) with respect to 100 parts by mass of the polymerizable monomer (A) are 170 to 270 parts by mass of the inorganic filler (B) and 5 to 50 parts by mass of the inorganic filler (C), respectively.
[2] The dental curable composition according to [1], in which a polarity of a zeta potential of the inorganic filler (B) measured in water is negative (minus).
[3] The dental curable composition according to [1] or [2], in which zeta potentials of the inorganic filler (B) and the inorganic filler (C) measured in water are -100 mV or more and -20 mV or less for the inorganic filler (B) and 10 mV or more and 60 mV or less for the inorganic filler (C), respectively.
[4] The dental curable composition according to any one of [1] to [3], in which a proportion of a mass of the inorganic filler (C) to a total mass of the inorganic filler (B) and the inorganic filler (C) is 1 to 20% by mass.

[5] The dental curable composition according to any one of [1] to [4], in which the polymerizable monomer (A) is composed of a (meth)acrylate-based polymerizable monomer, the inorganic filler (B) is composed of silica-based composite oxide particles, and the inorganic filler (C) is composed of crystalline rare earth metal fluoride particles, in which the particles have a full width at half maximum of a maximum intensity peak derived from the crystalline rare earth metal fluoride in an X-ray diffraction pattern of the particles of 0.3° or more.

[6] The dental curable composition according to [5], in which the silica-based composite oxide particles are at least one selected from the group consisting of silica-zirconia, silica-titania, silica-titania-barium oxide, and silica-titania-zirconia.

[7] The dental curable composition according to [5] or [6], in which the crystalline rare earth metal fluoride particles are at least one selected from the group consisting of ytterbium fluoride ($YbF_3$), lanthanum fluoride ($LaF_3$), cerium fluoride ($CeF_3$), and gadolinium fluoride ($GdF_3$).

[8] The dental curable composition according to any one of [5] to [7], in which the crystalline rare earth metal fluoride particles have a specific surface area increased by mechanochemical treatment.

[9] The dental curable composition according to any one of [1] to [8], which is used by being discharged from a syringe equipped with a needle tip.

[10] A flowable composite resin including the dental curable composition according to any one of [1] to [9].

Advantageous Effects of Invention

According to the present invention, a dental curable composition containing a polymerizable monomer and inorganic particles can be made to have a consistency suitable for discharging the dental curable composition from a needle tip without using inorganic particles surface-treated with a silane coupling agent having a long-chain alkylene group and without reducing the shapability. Moreover, the influence of the composition of the polymerizable monomer on such an effect is small, and even when, for example, a polymerizable monomer containing a large amount of hydrophilic polymerizable monomer is used, such a property can be reliably obtained.

Description of Embodiments

In the dental curable composition of the present invention, the polarity (sign: plus: "+" or minus: "-") of the zeta potential of the inorganic particles constituting the inorganic filler (B), which is blended in order to improve the physical properties of the cured product, is made constant (the same) among the constituent particles, and the inorganic filler (C) having a large specific surface area and different polarities of the constituent particles is used in combination, thereby making it possible to obtain an appropriate consistency without reducing the shapability without using inorganic particles surface-treated with a silane coupling agent having a long-chain alkylene group. The reason why such an effect is obtained is not necessarily clear, and the present invention is not bound by any theory, but the present inventors consider that the effect is obtained by the following mechanism.

That is, it is generally known that particles dispersed in a liquid have an electric charge, and when the electric charge is large, the dispersibility of the particles is increased by a repulsive force, and when the electric charge is close to zero, the aggregation property is increased. Further, it is known that fine particles having a large specific surface area such as fumed silica exhibit a thickening effect, but when the fine particles are blended in a blending amount giving a thickening effect necessary for obtaining the target shapability, the paste becomes too hard and an appropriate consistency cannot be obtained. In the present invention, the polarity of zeta potential is different between the constituent particles of the inorganic filler (B) blended to improve the strength and surface smoothness of the cured product and the constituent particles of the fine inorganic filler (C) having a large specific surface area blended to improve the viscosity, and therefore, it is considered that the static fluidity is lowered by forming a three dimensional network structure by electrostatic interaction of these particles, and the shapability is improved in spite of a small blending amount of the fine particles (without largely lowering the dynamic fluidity).

Hereinafter, the dental curable composition of the present invention will be described in detail, focusing on each component contained in the dental curable composition of the present invention. In the description herein, unless otherwise specified, the expression "x to y" using numerical values x and y means "x or more and y or less". In the event that only the numerical value y is indicated with a unit in such representation, such unit shall also apply to the numerical value x. Further, in the description herein, the term "(meth)acrylic" means both "acrylic" and "methacrylic". Similarly, the term "(meth)acrylate" means both "acrylate" and "methacrylate", and the term "(meth)acryloyl" means both "acryloyl" and "methacryloyl".

1. Regarding Polymerizable Monomer (A)

As the polymerizable monomer (A) in the dental curable composition of the present invention, polymerizable monomers used in conventional dental curable compositions, such as radically polymerizable monomers and cationically polymerizable monomers, can be used without particular limitation. Among these, it is preferable to use a (meth)acrylate-based polymerizable monomer which is generally used, specifically, an acidic group-containing (meth)acrylate-based polymerizable monomer, a hydroxy group-containing (meth)acrylate-based polymerizable monomer, or a monofunctional or polyfunctional (meth)acrylate-based polymerizable monomer having no substituent.

Examples of the (meth)acrylate-based polymerizable monomer which can be suitably used include the followings. That is, examples of the acidic group-containing (meth)acrylate-based polymerizable monomer include (meth)acrylic acid, N-(meth)acryloyl-p-aminobenzoic acid, 2-(meth)acryloyloxybenzoic acid, 2-(meth)acryloyloxyethyl phenyl hydrogen phosphate, and 2-(meth)acryloyloxyethyl phosphonic acid. Examples of the hydroxy group-containing (meth)acrylate-based polymerizable monomer include 2-hydroxyethyl (meth)acrylate, 3-hydroxypropyl (meth)acrylate, 4-hydroxybutyl (meth)acrylate, 6-hydroxyhexyl (meth)acrylate, 2,2-bis[(3-methacryloyloxy-2-hydroxypropyloxy)phenyl]propane, and 2,2-bis[4-(4-methacryloyloxy)-3-hydroxybutoxyphenyl]propane. Furthermore, examples of the monofunctional or polyfunctional (meth)acrylate-based polymerizable monomer having no substituent include methyl (meth)acrylate, ethyl (meth)acrylate, triethylene glycol dimethacrylate, tetraethylene glycol dimethacrylate, neopentyl glycol dimethacrylate, 1,6-hexanediol dimethacrylate, 1,9-nonanediol dimethacrylate, 1,6-bis(methacrylethyloxycarbonylamino)trimethylhexane, and 2,2-bis(4-methacryloyloxypolyethoxyphenyl)propane.

Among these polymerizable monomers, bifunctional or higher functional, more preferably bifunctional to tetrafunctional polymerizable monomers are preferable because of high polymerizability, and particularly high mechanical strength of the cured product. These polymerizable monomers may be used singly or as a mixture of different kinds thereof.

2. Regarding Inorganic Filler (B)

The inorganic filler (B) composed of inorganic particles, which is blended in the dental curable composition of the present invention, is an inorganic filler having an average particle diameter of 50 nm to 1 $\mu$m, and is blended in order to improve physical properties such as strength of a cured product. Here, the average particle diameter is an average particle diameter measured as follows using a scanning electron microscope or a transmission electron microscope, and means an average primary particle diameter. That is, the circular equivalent diameter (diameter of a circle having the same area as the area of the target particle): $X_i$ of each inorganic primary particle is obtained by performing image analysis on 30 or more inorganic primary particles which are randomly selected from a captured image by an electron microscope in which light and dark are clear and the outline of the particle can be discriminated, and the average particle diameter: X calculated by the following equation is obtained.

Average particle diameter :

$$X = \sqrt[3]{\frac{\sum_{i=1}^{n} X_i^3}{n}} \quad \text{(Average volume diameter)}$$

N: Number of particles observed

$X_i$: Particle diameter (diameter) of the i-th particle

When the average particle diameter of the inorganic filler (B) is less than 50 nm, the viscosity of the dental curable composition is increased and it becomes difficult to adjust the viscosity to a consistency suitable for discharge from a syringe equipped with a needle tip, and when the average particle diameter exceeds 1 $\mu$m, the polishability of the obtained cured product is decreased and a cured product with a smooth surface is difficult to obtain, and therefore the average particle diameter of the inorganic filler (B) needs to be 0.05 to 1 $\mu$m, and more preferably 0.1 to 0.8 $\mu$m.

The shape of the inorganic primary particles constituting the inorganic filler (B) is not particularly limited, and spherical, substantially spherical, or irregularly shaped particles can be used. However, from the viewpoint of excellent abrasion resistance and surface smoothness, spherical or substantially spherical particles are preferable. The term "substantially spherical" refers to a particle having an average uniformity degree of 0.6 or more, which is defined by the following equation based on the maximum length of each particle obtained by image analysis performed on the 30 or more n inorganic primary particles selected from a captured image in which the average particle diameter is measured as the major axis: $L_i$ and the minimum width: $B_i$ which is a diameter in a direction orthogonal to the major axis. The average uniformity degree is preferably 0.7 or more, and particularly preferably 0.8 or more. The upper limit value of the average uniformity degree is 1.

$$\text{Average uniformity degree} = \frac{\sum_{i=1}^{n} Bi/Li}{n}$$

In the dental curable composition of the present invention, in order to obtain the effect of the present invention, the polarity of the zeta potential of the inorganic filler (B) measured in water needs to be either negative (minus) or positive (plus), and needs to be constant between inorganic particles constituting the inorganic filler (B). Here, the polarity of the zeta potential measured in water means the polarity (sign, that is, plus or minus) of the zeta potential measured by an electrophoretic light scattering method for inorganic particles dispersed in ion-exchanged water at pH 7. When the polarity is different between the particles constituting the inorganic filler (B), it is considered that the electrostatic interaction with the particles constituting the inorganic filler (C) is unlikely to occur, thereby the effect of the present invention is unlikely to be obtained.

The polarity of the inorganic filler (B) may be either positive (plus) or negative (minus), but the polarity is preferably negative because the polarity of many silica-based particles used as an inorganic filler having an average particle diameter of 50 nm to 1 $\mu$m blended for the purpose of improving physical properties such as strength of a cured product is negative (minus).

The material of the inorganic particles constituting the inorganic filler (B) is not particularly limited as long as the polarity of the zeta potential is the same, and examples thereof include metal oxides such as amorphous silica, quartz, titania, zirconia, barium oxide, chromium oxide, iron oxide, and tungsten oxide; and composite oxides such as silica-zirconia, silica-titania, silica-titania-barium oxide, silica-titania-zirconia, borosilicate glass, aluminosilicate glass, and fluoroaluminosilicate glass, which are used as fillers in conventional dental curable compositions. For the reason that it is generally used and easily available, it is preferred to use a material having a negative polarity, in particular, a silica-based composite oxide such as silica-zirconia, silica-titania, silica-titania-barium oxide, or silica-titania-zirconia, and silica-zirconia mentioned above is more preferred.

Many of the silica-based composite oxide particles have strong acid points on the surface, and the negative degree of the zeta potential is also large in many cases. To be specific, the zeta potential in water is preferably -20 mV or less, and more preferably -40 mV or less. The lower limit of the zeta potential is not particularly limited, but is generally -100 mV or more. Note that the inorganic filler (B) may be a combination of a plurality of silica-based composite oxide particles having different average primary particle diameters, and in this case, the zeta potentials of the plurality of silica-based composite oxide particles are preferably within the above-mentioned ranges.

The inorganic filler (B) may be a mixture of inorganic particles made of different materials as long as the above conditions are satisfied, and these inorganic particles may be surface-treated with a silane coupling agent as long as the polarity of the zeta potential is not changed.

The blending amount of the inorganic filler (B) in the dental curable composition of the present invention needs to be 170 to 270 parts by mass with respect to 100 parts by mass of the polymerizable monomer (A). When the blending amount is less than 170 parts by mass, there is a risk that the physical properties (strength, abrasion resistance) of the cured product of the dental curable composition may be deteriorated, and when the blending amount exceeds 270 parts by mass, it becomes difficult to obtain a consistency suitable for discharge from a syringe equipped with a needle tip. From the viewpoint of achieving both high physical properties in the cured product and operability in a paste state, the blending amount of the inorganic filler (B) is preferably 190 to 260 parts by mass, and particularly preferably 200 to 250 parts by mass, with respect to 100 parts by mass of the polymerizable monomer (A).

The inorganic filler (B) may be blended as a so-called organic-inorganic composite filler compounded with a synthetic resin (a cured product of a polymerizable monomer). Known examples of the organic-inorganic composite filler include an organic-inorganic composite filler obtained by polymerizing a mixture of an inorganic filler, a polymerizable monomer, and a polymerization initiator and then pulverizing the mixture; and a microporous organic-inorganic composite filler obtained by immersing inorganic aggregated particles formed by aggregation of inorganic primary particles constituting an inorganic filler in a polymerizable monomer solvent containing a polymerizable monomer, a polymerization initiator, and an organic solvent, removing the organic solvent, and then polymerizing and curing the polymerizable monomer. The content of the inorganic filler in the organic-inorganic composite filler (hereinafter, also referred to as "filling rate") is usually 60 to 90% by mass.

When the inorganic filler (B) is blended as the organic-inorganic composite filler, the form of the organic-inorganic composite filler is not particularly limited as long as the filler is a composite of the inorganic filler (B) and a cured product of a polymerizable monomer, but it is preferable to blend an organic-inorganic composite filler in which the content (filling rate) of the inorganic filler (B) is 65 to 90% by mass, and particularly 70 to 90% by mass.

In addition, since the inorganic filler (B) in the organic-inorganic composite filler is coated with the synthetic resin, the inorganic filler (B) tends to become difficult to cause an interaction with the inorganic filler (C). The blending amount of the inorganic filler (B) is mainly determined from the viewpoint of the physical properties of the cured product (of the dental curable composition of the present invention), and it is difficult to determine the number and proportion of the constituent particles of the inorganic filler (B) that actually contribute to the above-mentioned interaction, but from the viewpoint of the obtained effect, when the inorganic filler (B) is blended as an organic-inorganic composite filler, it is preferable that a part of the inorganic filler (B) is blended as an organic-inorganic composite filler, and the proportion of the amount of the inorganic filler (B) blended as the organic-inorganic composite filler is preferably 70% by mass or less, and particularly 60% by mass or less, of the total amount (parts by mass), based on the mass of the inorganic filler (B). Specifically, it is preferable that the blending amount (parts by mass) of the organic-inorganic composite filler is an amount (parts by mass) calculated by {[the total amount (parts by mass) of the inorganic filler (B)] × [the above proportion (% by mass)] / [the filling rate (% by mass) of the inorganic filler (B) in the organic-inorganic composite filler]}.

3. Regarding Inorganic Filler (C)

The inorganic filler (C) composed of inorganic particles, which is blended in the dental curable composition of the present invention, is an inorganic filler having a polarity of a zeta potential measured in water opposite to the polarity of the inorganic filler (B) and having a specific surface area measured by a nitrogen gas adsorption method of 25 to 100 $m^2/g$. When the specific surface area is less than 25 $m^2/g$, even if the condition regarding the polarity of the zeta potential is satisfied, it is difficult to obtain the shapability in a paste state which is a feature of the present invention, and when it exceeds 100 $m^2/g$, the productivity decreases because a long time is required in the treatment step of increasing the specific surface area described later. From the viewpoint of the effect to be obtained, the specific surface area is preferably 25 to 80 $m^2/g$, and particularly preferably 30 to 70 $m^2/g$.

The average particle diameter of the inorganic filler (C) is usually 300 nm or less, and from the viewpoint of aesthetics (for example, polishability and transparency), is preferably 10 to 300 nm and more preferably 15 to 250 nm. Additionally, the refractive index of the inorganic filler (C) is preferably 1.4 to 1.7 from the viewpoint of transparency of the cured product of the dental curable composition. Note that the refractive index can be measured at 25°C by an Abbe refractometer.

In the dental curable composition of the present invention, in order to obtain the effect of the present invention, the polarity of the zeta potential of the inorganic filler (C) measured in water needs to be opposite to the polarity of the inorganic filler (B) and constant between inorganic particles constituting the inorganic filler (C). That is, when the polarity of the inorganic filler (B) is negative (or positive), the polarities of the inorganic particles constituting the inorganic filler (C) are all positive (or negative). The inorganic filler (C) may be a mixture of inorganic particles made of different materials, and these inorganic particles may be surface-treated with a silane coupling agent, as long as the conditions regarding the average particle diameter and the specific surface area and the conditions regarding the polarity are satisfied.

As described above, since the polarity of the zeta potential of the inorganic filler (B) is preferably negative (minus), the polarity of the inorganic filler (C) is preferably positive (plus). The absolute value of the zeta potential is not particularly limited, but the upper limit value thereof is generally 60 mV or less, and the lower limit value thereof is preferably 10 mV or more, and particularly preferably 20 mV or more.

As the inorganic particles in which the polarity of the zeta potential is positive (plus), rare earth metal compound particles are preferable from the viewpoint of color tone and safety. Among them, it is preferable to use crystalline rare earth metal fluoride particles such as ytterbium fluoride ($YbF_3$), lanthanum fluoride ($LaF_3$), cerium fluoride ($CeF_3$), and gadolinium fluoride ($GdF_3$), and it is most preferable to use ytterbium fluoride from the viewpoint of X-ray opacity. Whether or not it is crystalline can be determined by whether or not a peak based on a crystal plane is confirmed when X-ray diffraction measurement is performed.

The blending amount of the inorganic filler (C) in the dental curable composition of the present invention needs to be 5 to 50 parts by mass with respect to 100 parts by mass of the polymerizable monomer (A). When the blending amount is less than 5 parts by mass, it is difficult to obtain the shapability in a paste state, and when the blending amount is more than 50 parts by mass, there is a concern that the curability of the dental curable composition may decrease. From the viewpoint of easily obtaining shapability, curability, and a color tone or transparency suitable for the dental curable composition, the blending amount is preferably 7 to 45 parts by mass, and more preferably 10 to 40 parts by mass.

In addition, the mass proportion of the inorganic filler (C) to the total amount of the inorganic filler (B) and the inorganic filler (C) is preferably 1 to 20% by mass, and particularly preferably 2 to 15% by mass, for the reason that the flowability and sag of the dental curable composition of the present invention are suppressed, and good shapability is easily obtained.

4. Regarding Rare Earth Metal Fluoride Particles Suitable as Inorganic Filler (C)

Conventionally, industrially available rare earth metal fluoride particles have crystallinity, and the specific surface area thereof is less than 15 m$^2$/g even when the average particle diameter is small. Therefore, in order to use the rare earth metal fluoride particles as the inorganic filler (C), it is necessary to pulverize the industrially available crystalline rare earth metal fluoride particles to have a specific surface area in the range of 25 to 100 m$^2$/g before use. The pulverization method is not particularly limited, but it is preferable to use rare earth metal fluoride particles in which the specific surface area is increased by mechanochemical treatment and the amorphousness is enhanced at the same time, for the reason that a decrease in transparency when blended in a dental curable composition is suppressed and adjustment to transparency appropriate as a composite resin is facilitated.

Note that ytterbium fluoride (YbF$_3$) is well known as an X-ray contrast filler, and it is known that when it is blended in a dental curable composition such as a composite resin, the transparency of the cured product is decreased. Since it has been found for the first time by the present inventors that the above-mentioned decrease in transparency can be suppressed by enhancing the amorphousness by mechanochemical treatment, this point will be described in detail below.

The mechanochemical treatment means a treatment in which mechanical energy is applied to the raw material powder, and means a treatment in which at least one of mechanical grinding, pulverization, and dispersion is performed. From the viewpoint of reliably and efficiently increasing the specific surface area of the inorganic filler (C), it is preferable to adopt a wet method, and it is particularly preferable to perform a treatment using a wet beads mill.

The wet beads mill is a processing method in which a slurry obtained by mixing a powder to be processed and a medium is brought into contact with a medium (beads) that has been moved by stirring or vibration, thereby imparting a pulverization and disintegration action to the powder. Examples of a material used as a medium include glass, alumina, zircon, zirconia, steel, and resin, and it is preferable to use beads made of alumina or zirconia because the beads have excellent abrasion resistance and relatively little contamination. The size of the beads to be used may be selected according to the particle size of the target inorganic filler (C), and is not particularly limited, but in order to obtain inorganic particles preferable for blending into the dental curable composition, beads having a diameter of 0.01 to 0.5 mm are preferably used.

The wet bead mill is classified into a batch type in which a slurry and beads are directly charged into an apparatus and treated, a circulation type in which a slurry is circulated between a tank and an apparatus, and a pass type in which a slurry is passed through an apparatus a predetermined number of times, depending on the operation method, and these operation methods may be selected depending on the amount of inorganic particles used for the treatment. It is preferable to use a circulation type bead mill because it has good productivity and can treat a relatively large amount of inorganic particles.

Depending on the operation method such as the circulation type or the pass type, it is necessary to separate the slurry and the beads when the mechanochemical treatment is carried out. Examples of the bead separation method include a slit type, a screen type, and a centrifugal separation type. These bead separation methods may be selected depending on the particle diameter of the beads to be used, and any method can be used without particular limitation. The concentration of the slurry used for the mechanochemical treatment is preferably 50 parts by mass or less of the inorganic particles with respect to 100 parts by mass of the medium. When the inorganic particles in the slurry exceed 50 parts by mass, the viscosity of the slurry increases, and it becomes difficult to perform the mechanochemical treatment.

The slurry viscosity increase can be suppressed by adding a dispersant to the slurry, and a slurry having a higher concentration can be subjected to mechanochemical treatment. The dispersant to be used is not particularly limited as long as it is a surfactant used in an ordinary filler, and examples thereof include a nonionic surfactant, an anionic surfactant, a cationic surfactant, an ampholytic surfactant, and a polymer surfactant thereof. Specific examples thereof include a glycerin fatty acid ester and an alkylene glycol adduct thereof, an aliphatic monocarboxylic acid salt, an alkylamine salt, and an alkyl betaine. When added to the dental composition, a cationic surfactant is preferably used from the viewpoint of dispersibility after mechanochemical treatment.

The mechanochemical treatment conditions vary depending on the conditions such as the operation method of the wet beads mill used, the bead diameter, the type of the inorganic filler (C), and the slurry concentration. The adjustment of these conditions can be performed by performing a preliminary experiment in the apparatus and conditions for actually performing the mechanochemical treatment, and confirming the specific surface area of the treated inorganic filler (C) with respect to the mechanochemical treatment time.

In the case where the inorganic filler (C) is obtained by treating crystalline rare earth metal fluoride particles, in addition to the effect of increasing the specific surface area by the aforementioned mechanochemical treatment, the crystallinity of the crystalline rare earth metal fluoride particles decreases, the decrease in transparency when blended in the dental curable composition is suppressed, and adjustment to transparency appropriate as a composite resin is facilitated.

The crystallinity of the crystalline rare earth metal fluoride particles can be evaluated by the full width at half maximum of the maximum intensity peak derived from the crystalline rare earth metal fluoride in an X-ray diffraction pattern, and the crystallinity is higher as the full width at half maximum is smaller. Incidentally, the full width at half maximum of the maximum intensity peak of rare earth metal fluorides generally used as X-ray opaque materials and commercially available rare earth metal fluoride powders that can be obtained as raw material powders is usually less than 0.3° (specifically, about 0.12° to 0.27°), and when such a rare earth metal fluoride powder is blended, the transparency is decreased. In order to obtain the effect of suppressing the decrease in transparency, it is necessary to set the full width at half maximum of the maximum intensity peak derived from the crystalline rare earth metal fluoride in the X-ray diffraction pattern to 0.3° or more by performing, for example, mechanochemical treatment, and from the viewpoint of suppressing the decrease in transparency, the full width at half maximum of the maximum intensity peak is preferably 0.4° or more, and particularly preferably 0.5° or more.

The full width at half maximum of the maximum intensity peak increases as the mechanochemical treatment time increases, but the amount of increase varies depending on various conditions, and therefore it is preferable to perform preliminary experiments in the same manner as described above and confirm the full width at half maximum of the treated crystalline rare earth metal fluoride particles with respect to the mechanochemical treatment time. Note that the upper limit value of the full width at half maximum is not particularly limited, but when the crystalline rare earth metal fluoride particles are subjected to mechanochemical treatment, the full width at half maximum usually does not exceed 2.0°.

The full width at half maximum of the maximum intensity peak in the X-ray diffraction pattern can be determined by performing X-ray diffraction measurement on the inorganic filler (C) composed of crystalline rare earth metal fluoride particles in the present invention. Specifically, the full width at half maximum can be determined by performing X-ray diffraction measurement in the range of $2\theta$; 20 to 120° with an X-ray diffraction apparatus, identifying the peak derived from the crystalline rare earth metal fluoride in the obtained X-ray diffraction pattern (chart) having $2\theta$ (°) on the horizontal axis and diffraction intensity on the vertical axis, and calculating the full width at half maximum of the peak having the maximum intensity (for example, for $YbF_3$, a peak corresponding to the crystal plane (1,1,1) appearing in the vicinity of $2\theta = 28.0°$), that is, the width of the peak at the intensity at which the intensity is 50% of the peak intensity (maximum intensity) (the absolute value of the difference of $2\theta$ between two intersection points of the intensity and the peak line: unit "deg [°]"). In the measurement, it is preferable to use, as a measurement sample, a powder from which coarse grains are removed by, for example, using a sieve having an opening of 100 $\mu$m.

5. Polymerization Initiator

A polymerization initiator may be added to the dental curable composition of the present invention. The polymerization initiator is not particularly limited as long as it has a function of polymerizing the polymerizable monomer (A), but it is preferable to use a photopolymerization initiator or a chemical polymerization initiator used in dental direct filling and restoration applications in which curing is often performed in the oral cavity, and it is more preferable to use a photopolymerization initiator (composition) from the viewpoint of simplicity without the need for a mixing operation.

As the polymerization initiator used for photopolymerization, benzoin alkyl ethers such as benzoin methyl ether, benzoin ethyl ether, and benzoin isopropyl ether; benzil ketals such as benzil dimethyl ketal and benzil diethyl ketal; benzophenones such as benzophenone, 4,4'-dimethylbenzophenone, and 4-methacryloxybenzophenone; $\alpha$-diketones such as diacetyl, 2,3-pentanedione benzyl, camphorquinone, 9,10-phenanthraquinone, and 9,10-anthraquinone; thioxanthone compounds such as 2,4-diethoxythioxanthone, 2-chlorothioxanthone, and methylthioxanthone; and bisacylphosphine oxides such as bis-(2,6-dichlorobenzoyl)phenylphosphine oxide, bis-(2,6-dichlorobenzoyl)-2,5-dimethylphenylphosphine oxide, bis-(2,6-dichlorobenzoyl)-4-propylphenylphosphine oxide, bis-(2,6-dichlorobenzoyl)-1-naphthylphosphine oxide, and bis (2,4,6-trimethylbenzoyl)-phenylphosphine oxide can be used.

In addition, a reducing agent is often added to the photopolymerization initiator, and examples thereof include tertiary amines such as 2-(dimethylamino)ethyl methacrylate, ethyl 4-dimethylaminobenzoate, and N-methyldiethanolamine; aldehydes such as lauryl aldehyde, dimethylaminobenzaldehyde, and terephthalaldehyde; and sulfur-containing compounds such as 2-mercaptobenzoxazole, 1-decanethiol, thiosalicylic acid, and thiobenzoic acid.

Further, in many cases, a photoacid generator is used in addition to the photopolymerization initiator and the reducing agent. Examples of such a photoacid generator include a diaryliodonium salt-based compound, a sulfonium salt-based compound, a sulfonic acid ester compound, a halomethyl-substituted-S-triazine derivative, and a pyridinium salt-based compound.

These polymerization initiators may be used alone or as a mixture of two or more thereof. The blending amount of the polymerization initiator may be selected as an effective amount depending on the purpose, and is usually the proportion of 0.01 to 10 parts by mass, and more preferably the proportion of 0.1 to 5 parts by mass with respect to 100 parts by mass of the polymerizable monomer.

6. Other Additives

To the dental curable composition of the present invention, other inorganic fillers other than the inorganic fillers (B) and (C), specifically, those that do not satisfy the conditions regarding the average particle diameter and the specific surface area in (B) and (C), can also be added to the extent that the effects of the present invention are not impaired. Furthermore, in the dental curable composition of the present invention, additives such as a polymerization inhibitor, a pigment, an ultraviolet absorber, and a fluorescent agent can be blended within a range that does not impair the effects.

The dental curable composition of the present invention can be made to have a consistency suitable for discharge from a needle tip without using inorganic particles surface-treated with a silane coupling agent having a long-chain alkylene group and without reducing the shapability. Since the inorganic particles surface-treated with a silane coupling agent having a long-chain alkylene group have high hydrophobicity as described above, the inorganic particles are easily restricted in terms of production method and tend to have low affinity with hydrophilic polymerizable monomers, and therefore, the blending amount of the inorganic particles is preferably small, and more preferably, the inorganic particles are not blended.

Therefore, in the dental curable composition of the present invention, the content of the inorganic filler surface-treated with a silane coupling agent having a long-chain alkylene group can be set to preferably 50 parts by mass or less, more preferably 10 parts by mass or less, and still more preferably 0 part by mass, with respect to 100 parts by mass of the polymerizable monomer (A). Note that the long-chain alkylene group means an alkylene group having 6 or more carbon atoms, and is generally an alkylene group having 6 or more and 13 or less carbon atoms.

7. Regarding Method for Preparing Dental Curable Composition of the Present Invention and Use Thereof

The dental curable composition of the present invention can be prepared by sufficiently kneading a predetermined amount of each component and each optional component to be added as necessary to obtain a paste, and then defoaming the paste under reduced pressure to remove air bubbles.

As described above, the dental curable composition of the present invention has a consistency suitable for discharge from a syringe equipped with a needle tip, has low fluidity (flowability and sag), and has appropriate shapability, and thus is particularly suitable as a flowable composite resin.

In addition, as described above, the consistency is an index indicating the hardness (softness) of the fluid paste, but in the present invention, in consideration of the fluidity, 0.2 g of a paste-like dental curable composition (hereinafter, also simply referred to as a paste) is crushed at 25°C with a load of 50 g, and the long diameter and the short diameter of the paste are measured after 10 seconds have elapsed, and the average value of both is quantitatively evaluated. When the value of the consistency is smaller than that of 10 mm, the discharge force at the time of discharge from a syringe becomes high, and extrusion becomes difficult. On the other hand, when it is larger than 20 mm, the flowability and sag of the paste described later become large, and it becomes difficult to obtain the shapability. Thus, when the value of the consistency is 10 to 20 mm, it can be said that a paste property is obtained, which is easy to use when discharging from a syringe through a needle, but 12 to 18 mm is more preferable.

In the present invention, the "flowability" is evaluated as a value obtained by discharging the paste of 0.1 g from a syringe equipped with a 20G needle tip onto a glass plate in a circle having a 5 mm radius and measuring the spread of the paste after 2 minutes at 37°C, and the "sag" is evaluated as a value obtained by weighing the paste of 0.03 g from a syringe equipped with a 20G needle tip onto a glass plate, fixing the paste so that the dental curable composition is vertical, and measuring the movement of the paste after 1 minute at 37°C. In a case where the flowability is 8 mm or less, since the shape of the paste discharged through the needle when used in the oral cavity is maintained, the paste has shapability and is easy to build up and modify the shape. For the reason that building up is easier, the flowability is preferably 7 mm or less and more preferably 6 mm or less. Further, in a case where the sag is 2 mm or less, since the paste discharged through the needle when used in the oral cavity stays in the restored part of the tooth, the paste is easy to use, and 1 mm or less is more preferable.

Examples

Hereinafter, the present invention will be more specifically described by Examples, but the present invention is not limited to these Examples.

First, in Examples and Comparative Examples, substances used as raw materials of the compositions to be prepared and their abbreviations, and evaluation methods of the above-mentioned raw materials and prepared compositions will be described.

1. Raw Materials and Their Abbreviations

(1) Polymerizable monomers

- UDMA: 1,6-bis(methacrylethyloxycarbonylamino)-2,2-4-trimethylhexane
- GMA: 2,2-bis[(3-methacryloyloxy-2-hydroxypropyloxy)phenyl]propane
- 3G: triethylene glycol dimethacrylate
- D-2.6E: 2,2-bis(4-methacryloyloxypolyethoxyphenyl)propane

(2) Inorganic particles for inorganic filler

The average primary particle diameter and the average uniformity degree in each of the particles shown below are values determined based on an evaluation method described later with respect to a filler (powder) formed of each of the particles.

(2)-1 Silica-based composite oxide particles

·F-1: spherical silica-zirconia particles having an average primary particle diameter of 150 nm produced by a sol-gel method (average uniformity degree: 0.95, refractive index: 1.52)
·F-2: spherical silica-zirconia particles having an average primary particle diameter of 280 nm produced by a sol-gel method (average uniformity degree: 0.95, refractive index: 1.52)
·F-3: spherical silica-zirconia particles having an average primary particle diameter of 400 nm produced by a sol-gel method (average uniformity degree: 0.90, refractive index: 1.52)
·F-4: spherical silica-zirconia particles having an average primary particle diameter of 70 nm produced by a sol-gel method (average uniformity degree: 0.90, refractive index: 1.52)
·F-5: irregularly shaped silica-zirconia particles having an average primary particle diameter of 1000 nm produced by a sol-gel method (refractive index: 1.52)

(2) -2 Crystalline rare earth metal fluoride particles

·YbF3-40: ytterbium trifluoride having an average primary particle diameter of 46 nm (manufactured by Sukgyung AT Co., Ltd.)
·YbF3-100: ytterbium trifluoride having an average primary particle diameter of 106 nm (manufactured by Sukgyung AT Co., Ltd.)
·YbF3-300: ytterbium trifluoride having an average primary particle diameter of 270 nm (manufactured by Sukgyung AT Co., Ltd.)
·CeF3: cerium trifluoride having an average primary particle diameter of 354 nm (manufactured by FUJIFILM Wako Pure Chemical Corporation)
·GdF3: gadolinium trifluoride having an average primary particle diameter of 389 nm (manufactured by FUJIFILM Wako Pure Chemical Corporation)
·LaF3: lanthanum trifluoride having an average primary particle diameter of 403 nm (manufactured by FUJIFILM Wako Pure Chemical Corporation)

Note that the refractive index of YbF3 is 1.55, the refractive index of CeF3 is 1.63, the refractive index of GdF3 is 1.62, and the refractive index of LaF3 is 1.58.

(3) Polymerization initiator

·CQ: camphorquinone
·DMBE: ethyl N,N-dimethyl-p-benzoate

2. Evaluation Method and Evaluation Result of Inorganic Filler

(1) Filler composed of silica-based composite oxide particles

The average primary particle diameter and the zeta potential of the five types of inorganic fillers each consisting of only F-1 to F-5 were determined as follows. The results are shown in Table 1.

<Method for Measuring Average Primary Particle Diameter>

A photograph of the powder was taken at a magnification of 5000 to 100000 times using a scanning electron microscope ("XL-30S" manufactured by Philips), and the image was processed using image analysis software ("IP-1000PC" manufactured by Asahi Kasei Engineering Corporation), and the average primary particle diameter was determined based on the measured value of the number (30 or more) of particles observed in the unit field of view of the photograph.

<Method for Measuring Zeta Potential of Inorganic Particles>

Inorganic particles were suspended in ion-exchanged water having a pH of 7, and dispersed in water by being irradiated with ultrasonic waves for 30 minutes so that the concentration of the suspended water was 1.0% by mass. The zeta potential of this suspended water was measured using a zeta potential measuring instrument ("ELSZ-2000" manufactured by Otsuka Electronics Co., Ltd.). The measurement was performed three times for each sample, and the average thereof was defined as the zeta potential.

<Method for Measuring Refractive Index of Inorganic Particles>

The refractive index of the inorganic particles was measured by an immersion method using an Abbe refractometer (manufactured by Atago Co., Ltd.) (measuring wavelength: 589 nm). That is, 1 g of the inorganic particles was dispersed in 50 mL of anhydrous toluene in a 100 mL sample bottle in a thermostatic chamber at 25°C, 1-bromotoluene was added dropwise little by little while stirring with a stirrer, the refractive index of the dispersion liquid at the time when the dispersion liquid became most transparent was measured, and the obtained value was taken as the refractive index of the inorganic particles.

Table 1

| Abbreviation | Material | Average primary particle diameter (nm) | Shape | Zeta potential (mV) |
|---|---|---|---|---|
| F-1 | Silica zirconia | 150 | Spherical | -44 |
| F-2 | Silica zirconia | 280 | Spherical | -40 |
| F-3 | Silica zirconia | 400 | Spherical | -38 |
| F-4 | Silica zirconia | 70 | Spherical | -42 |
| F-5 | Silica zirconia | 1000 | Irregularly shaped | -32 |

(2) Filler composed of rare earth metal fluoride particles

Each of the crystalline rare earth metal fluoride particles (YbF3-40, YbF3-100, YbF3-300, CeF3, GdF3, and LaF3) was subjected to mechanochemical treatment to prepare seven types of inorganic fillers: MF-1C, MF-2C, MF-3C, MF-4C, MF-5C, MF-6C, and MF-7C as the inorganic filler (C). In addition, the six types of crystalline rare earth metal fluoride particles having a specific surface area not satisfying the conditions of the inorganic filler (C) in the present invention without performing mechanochemical treatment were used as inorganic fillers (C'): MF-1, MF-2, MF-3, MF-5, MF-6 and MF-7. The mechanochemical treatment was performed by subjecting a slurry obtained by mixing 5.0 parts by mass of each of the crystalline rare earth metal fluoride particles with 100 parts by mass of ion-exchanged water to dispersion treatment using a wet beads mill SC50 (manufactured by Nippon Coke & Engineering Co., Ltd.) at a rotation speed of 3000 rpm for a treatment time shown in Table 2 using 100 g of 0.3 mmφ zirconia beads as a medium. The obtained slurry was dried under reduced pressure using an evaporator to obtain a white solid. The obtained solid was pulverized in a mortar, and then coarse particles were removed by a sieve having an opening of 100 μm to obtain a powder as various inorganic fillers.

Regarding the inorganic filler thus obtained, the average primary particle diameter and zeta potential were measured in the same manner as in the above (1), and the specific surface area, 2θ of the peak of the crystal plane (1,1,1) in the X-ray diffraction pattern, and the full width at half maximum (deg: °) were measured as follows. The results are shown in Table 2.

Table 2-1

| Abbreviation | Crystalline rare earth metal fluoride particles | Treatment time (min.) | Average primary particle diameter (nm) |
|---|---|---|---|
| MF-1 | YbF3-40 | 0 | 46 |
| MF-2 | YbF3-100 | | 106 |
| MF-3 | YbF3-300 | | 270 |
| MF-1C | YbF3-40 | 180 | 44 |
| MF-2C | YbF3-100 | | 90 |
| MF-3C | YbF3-300 | | 240 |
| MF-4C | YbF3-40 | 600 | 15 |

Table 2-1 Continued

| Abbreviation | Zeta potential (mV) | Specific surface area (m$^2$/g) | Maximum peak: (1,1,1) | |
|---|---|---|---|---|
| | | | 2θ (°) | Full width at half maximum (°) |
| MF-1 | +49 | 14 | 28.0 | 0.17 |
| MF-2 | +49 | 11 | 28.0 | 0.20 |
| MF-3 | +48 | 6 | 27.9 | 0.22 |
| MF-1C | +50 | 48 | 27.9 | 0.43 |
| MF-2C | +49 | 39 | 28.0 | 0.42 |
| MF-3C | +49 | 27 | 28.0 | 0.39 |
| MF-4C | +50 | 62 | 27.9 | 0.77 |

Table 2-2

| Abbreviation | Crystalline rare earth metal fluoride particles | Treatment time (min.) | Average primary particle diameter (nm) |
|---|---|---|---|
| MF-5 | CeF3 | 0 | 354 |
| MF-6 | GdF3 | | 389 |
| MF-7 | LaF3 | | 403 |
| MF-5C | CeF3 | 180 | 231 |
| MF-6C | GdF3 | | 256 |
| MF-7C | LaF3 | | 273 |

Table 2-2 Continued

| Abbreviation | Zeta potential (mV) | Specific surface area (m$^2$/g) | Maximum peak: (1,1,1) | |
|---|---|---|---|---|
| | | | 2θ (°) | Full width at half maximum (°) |
| MF-5 | +34 | 6 | 27.8 | 0.13 |

(continued)

| Abbreviation | Zeta potential (mV) | Specific surface area (m²/g) | Maximum peak: (1,1,1) | |
|---|---|---|---|---|
| | | | 2θ (°) | Full width at half maximum (°) |
| MF-6 | +44 | 4 | 27.6 | 0.17 |
| MF-7 | +21 | 1 | 27.7 | 0.12 |
| MF-5C | +35 | 49 | 27.8 | 0.42 |
| MF-6C | +46 | 47 | 27.6 | 0.58 |
| MF-7C | +25 | 50 | 27.7 | 0.41 |

<Method for Measuring Specific Surface Area>

0.1 g of the above-described inorganic filler composed of various inorganic particles was placed in a sample cell, and pre-treatment was performed by vacuum evacuation at 100°C for 3 hours using a pre-treatment apparatus ("BELPREP - miniII" manufactured by MicrotracBEL Corp.). Then, a nitrogen adsorption isotherm was obtained by a gas adsorption method pore distribution measurement apparatus ("BELSORP-miniII" manufactured by MicrotracBEL Corp.) using nitrogen as an adsorption gas and liquid nitrogen as a refrigerant, and a specific surface area was calculated by the BET method.

<Method for Measuring 2θ and Full Width at Half Maximum (deg: °) of Crystal Plane (1,1,1)>

A sample stage was filled with various fillers, and 2θ and full width at half maximum (deg: °) of a peak of the crystal plane (1,1,1) were read from an X-ray diffraction pattern (chart) obtained by performing measurement using an X-ray diffraction apparatus {"Smartlab" manufactured by Rigaku Corporation}, in which the horizontal axis represents 2θ (°) and the vertical axis represents diffraction intensity.

3. Examples and Comparative Examples

(Example 1)

A polymerizable monomer solution was prepared by completely dissolving 0.20 parts by mass of CQ and 0.35 parts by mass of DMBE as polymerization initiators in a polymerizable monomer composed of 80 parts by mass of UDMA and 20 parts by mass of 3G. Thereafter, 160 parts by mass of F-3 surface-treated with γ-methacryloyloxypropyltrimethoxysilane, 68 parts by mass of F-4 surface-treated with γ-methacryloyloxypropyltrimethoxysilane, 12 parts by mass of MF-1C, and the polymerizable monomer solution were mixed until it became homogeneous in a mortar, and then defoamed to prepare a paste-like dental curable composition.

With respect to the obtained dental curable composition, the consistency, flowability, and sag in a paste state, the bending strength of the cured product, and the contrast ratio of the cured product were evaluated by the following methods. The results were shown in Table 3.

<Method for Measuring "Consistency">

The prepared past-like dental curable composition was allowed to stand in an incubator at 45°C for 1 day, and then the paste allowed to stand at 25°C for 30 minutes was measured for consistency by the following method. 0.2 g of the paste was weighed onto polypropylene film with a raised center. Polypropylene film, a glass plate, and a weight (50 g in total) were placed thereon in this order, and after 10 seconds had passed, the glass plate and the weight were removed, and the major axis and the minor axis of the paste were measured through the polypropylene film, and the average of both was calculated. The above evaluation was performed twice, and the average value was taken as the consistency of the paste. A case where the consistency was 10 to 20 mm was taken as the acceptance criterion of the evaluation.

<Method for Measuring "Sag">

The prepared paste-like dental curable composition was allowed to stand in an incubator at 45°C for 1 day, and then filled into a cylindrical syringe. A plunger for extruding the content of the syringe and a 20G needle tip to be attached to the tip of the syringe were attached. Thereafter, the paste was allowed to stand in a thermostatic chamber at 25°C for 30 minutes, and then 0.03 g of the dental curable composition was extruded from the tip of a needle tip onto a glass plate. The discharged dental curable composition was fixed so as to be vertical, and the paste was allowed to stand in an incubator at 37°C for 1 minute, and the distance by which the paste moved was measured. The above evaluation was performed twice, and the average value was taken as the sag of the paste. A case where the sag was 2 mm or less was taken as the acceptance criterion of the evaluation.

<Method for Measuring "Flowability">

The prepared paste-like dental curable composition was allowed to stand in an incubator at 45°C for 1 day, and then filled into a cylindrical syringe. A plunger for extruding the content of the syringe and a 20G needle tip to be attached to the tip of the syringe were attached. Thereafter, the paste was allowed to stand in a thermostatic chamber at 25°C for 30 minutes, a circle having a diameter of 5 mm was drawn on the glass plate in advance, 0.1 g of the dental curable composition was discharged into the circle, and the paste was allowed to stand in a horizontal state for 2 minutes in an incubator at 37°C. Regarding the spread of the paste at that time, the major axis and the minor axis were measured, and the average of both was calculated. The above evaluation was performed twice, and the average value was taken as the flowability of the paste. A case where the flowability was 8 mm or less was taken as the acceptance criterion of the evaluation.

<Method for Measuring Bending Strength>

The paste of the dental curable composition was filled in a stainless steel mold, and in a state of being pressed against polypropylene film, light irradiation was performed by bringing the paste into close contact with the polypropylene film from one surface thereof three times for 30 seconds by using a visible light irradiator ("Power Light" manufactured by Tokuyama Corporation) at different positions so that the entire surface was irradiated with light. Next, the opposite surface was also brought into close contact with the polypropylene film in the same manner and irradiated with light three times for 30 seconds to obtain a cured product. The cured product was shaped into a prismatic shape of $2 \times 2 \times 25$ mm using #1500 water resistant abrasive paper, and this sample piece was attached to a testing machine ("Autograph AG5000D" manufactured by Shimadzu Corporation). The three point bending fracture strength was measured at an inter-fulcrum distance of 20 mm and a crosshead speed of 1 mm/min, a load-deflection curve was obtained, and the bending strength was obtained from the following equation. The evaluation was performed on five test pieces, and the average value was defined as the bending strength.

$$\text{Equation: } \sigma_B = (3PS)/(2WB^2)$$

Note that the symbols in the above equation represent: $\sigma_B$: bending strength (Pa), P: load at test piece breakage (N), S: inter-fulcrum distance (m), W: width of test piece (m), B: thickness of test piece (m), respectively.

<Evaluation Method of Contrast Ratio (Yb/Yw) of Curable Composition Cured Product>

The composition prepared in each of Examples and Comparative Examples was placed in a mold having a through hole of 7 mm$\varphi \times$ 1 mm, and polyester film was pressed against both surfaces. Both surfaces were irradiated with light for 30 seconds each with a visible light irradiator ("Power Light" manufactured by Tokuyama Corporation) to be cured, and then the cured product was taken out from the mold and the Y values (background colors black and white) of the tristimulus values of the cured product were measured using a color difference meter ("TC-1800MKII" manufactured by Tokyo Denshoku Co., Ltd.). The contrast ratio (Yb/Yw) was calculated based on the following formula:

Contrast ratio (Yb/Yw) = (Y value when background color is blank)/(Y value when background color is white).

Note that the contrast ratio (Yb/Yw) is an index of transparency, and a lower value means higher transparency.

(Examples 2 to 15 and Comparative Examples 1 to 7)

A past-like dental curable composition was prepared in the same manner as in Example 1 except that the types and blending amounts of the inorganic filler and the polymerizable monomer to be used were changed as shown in Table 3, Table 4, and Table 5, and the obtained composition and a cured product thereof were evaluated in the same manner as in Example 1. The results are shown in Table 3, Table 4, and Table 5.

Table 3

| | | Example 1 | Example 2 | Example 3 | Example 4 |
|---|---|---|---|---|---|
| Inorganic filler (B) (parts by mass) | F-1 | | | | |
| | F-2 | | | | |
| | F-3 | 160 | 160 | 160 | 165 |
| | F-4 | 68 | 68 | 68 | 70 |
| | F-5 | | | | |
| Inorganic filler (C) (parts by mass) | MF-1C | 12 | 12 | 12 | 5 |
| Polymerizable monomer (A) (parts by mass) | UDMA | 80 | | 30 | 80 |
| | GMA | | 60 | | |
| | 3G | 20 | 40 | 20 | 20 |
| | D-2.6E | | | 50 | |
| Consistency (mm) | | 16.0 | 17.0 | 13.0 | 18.0 |
| Flowability (mm) | | 5.0 | 5.5 | 5.0 | 7.0 |
| Sag (mm) | | 0.0 | 1.0 | 0.0 | 1.5 |
| Bending strength (MPa) | | 150 | 152 | 148 | 155 |
| Contrast ratio Yb/Yw | | 0.28 | 0.40 | 0.35 | 0.27 |

Table 3 (Continued)

| | | Example 5 | Example 6 | Example 7 | Example 8 |
|---|---|---|---|---|---|
| Inorganic filler (B) (parts by mass) | F-1 | | | 190 | |
| | F-2 | | | | 190 |
| | F-3 | 134 | | | |
| | F-4 | 58 | | | |
| | F-5 | | 190 | | |
| Inorganic filler (C) (parts by mass) | MF-1C | 48 | 10 | 10 | 10 |
| Polymerizable monomer (A) (parts by mass) | UDMA | 80 | 80 | 80 | 80 |
| | GMA | | | | |
| | 3G | 20 | 20 | 20 | 20 |
| | D-2.6E | | | | |
| Consistency (mm) | | 12.0 | 20.0 | 16.0 | 17.0 |
| Flowability (mm) | | 4.0 | 6.0 | 5.0 | 5.2 |
| Sag (mm) | | 0.0 | 1.5 | 0.0 | 0.5 |
| Bending strength (MPa) | | 138 | 150 | 142 | 140 |
| Contrast ratio Yb/Yw | | 0.30 | 0.28 | 0.28 | 0.28 |

Table 4

| | | Example 9 | Example 10 | Example 11 | Example 12 |
|---|---|---|---|---|---|
| Inorganic filler (B) (parts by mass) | F-3 | 160 | 160 | 160 | 186 |
| | F-4 | 68 | 68 | 68 | 80 |
| | F-5 | | | | |
| Inorganic filler (C) (parts by mass) | MF-1C | | | | 14 |
| | MF-2C | 12 | | | |
| | MF-3C | | 12 | | |
| | MF-4C | | | 12 | |
| Inorganic filler (C') (parts by mass) | MF-1 | | | | |
| Polymerizable monomer (A) (parts by mass) | UDMA | 80 | 80 | 80 | 80 |
| | 3G | 20 | 20 | 20 | 20 |
| Consistency (mm) | | 16.0 | 17.0 | 16.0 | 15.0 |
| Flowability (mm) | | 5.5 | 6.0 | 4.8 | 4.8 |
| Sag (mm) | | 0.0 | 0.5 | 0.0 | 0.0 |
| Bending strength (MPa) | | 148 | 145 | 152 | 155 |
| Contrast ratio Yb/Yw | | 0.28 | 0.28 | 0.28 | 0.28 |

Table 4 (Continued)

| | | Comparative Example 1 | Comparative Example 2 | Comparative Example 3 | Comparative Example 4 |
|---|---|---|---|---|---|
| Inorganic filler (B) (parts by mass) | F-3 | 168 | | 160 | 134 |
| | F-4 | 72 | | 68 | 58 |
| | F-5 | | 200 | | |
| Inorganic filler (C) (parts by mass) | MF-1C | | | | |
| | MF-2C | | | | |
| | MF-3C | | | | |
| | MF-4C | | | | |
| Inorganic filler (C') (parts by mass) | MF-1 | | | 12 | 48 |
| Polymerizable monomer (A) (parts by mass) | UDMA | 80 | 80 | 80 | 80 |
| | 3G | 20 | 20 | 20 | 20 |
| Consistency (mm) | | 20.0 | 22.0 | 20.0 | 9.0 |
| Flowability (mm) | | 9.0 | 11.0 | 8.5 | 5.8 |
| Sag (mm) | | 3.0 | 9.0 | 3.0 | 0.0 |
| Bending strength (MPa) | | 151 | 145 | 130 | 90 |
| Contrast ratio Yb/Yw | | 0.28 | 0.28 | 0.38 | 0.58 |

Table 5

| | | Example 13 | Example 14 | Example 15 |
|---|---|---|---|---|
| Inorganic filler (B) (parts by mass) | F-3 | 160 | 160 | 160 |
| | F-4 | 68 | 68 | 68 |
| Inorganic filler (C) (parts by mass) | MF-5C | 12 | | |
| | MF-6C | | 12 | |
| | MF-7C | | | 12 |
| Inorganic filler (C') (parts by mass) | MF-5 | | | |
| | MF-6 | | | |
| | MF-7 | | | |
| Polymerizable monomer (A) (parts by mass) | UDMA | 80 | 80 | 80 |
| | 3G | 20 | 20 | 20 |
| Consistency (mm) | | 16.0 | 16.0 | 15.5 |
| Flowability (mm) | | 5.0 | 5.0 | 4.8 |
| Sag (mm) | | 0.0 | 0.0 | 0.0 |
| Bending strength (MPa) | | 151 | 152 | 150 |
| Contrast ratio Yb/Yw | | 0.34 | 0.33 | 0.30 |

Table 5 (Continued)

| | | Comparative Example 5 | Comparative Example 6 | Comparative Example 7 |
|---|---|---|---|---|
| Inorganic filler (B) (parts by mass) | F-3 | 160 | 160 | 160 |
| | F-4 | 68 | 68 | 68 |
| Inorganic filler (C) (parts by mass) | MF-5C | | | |
| | MF-6C | | | |
| | MF-7C | | | |
| Inorganic filler (C') (parts by mass) | MF-5 | 12 | | |
| | MF-6 | | 12 | |
| | MF-7 | | | 12 |
| Polymerizable monomer (A) (parts by mass) | UDMA | 80 | 80 | 80 |
| | 3G | 20 | 20 | 20 |
| Consistency (mm) | | 19.0 | 20.0 | 20.0 |
| Flowability (mm) | | 8.9 | 9.0 | 9.2 |
| Sag (mm) | | 3.0 | 3.0 | 4.0 |
| Bending strength (MPa) | | 135 | 131 | 128 |
| Contrast ratio Yb/Yw | | 0.41 | 0.41 | 0.39 |

As can be seen from the results of Examples 1 to 15, the dental curable composition of the present invention has a consistency suitable for being discharged from a syringe equipped with a needle tip regardless of the type of the matrix monomer or the inorganic particles, the fluidity (flowability and sag) of the paste is suppressed, and the shapability and operability suitable for a flowable composite resin are exhibited.

As can be seen from the results of Comparative Example 1 and Comparative Example 2, when the inorganic filler (C) is not contained, the consistency and the fluidity (flowability and sag) are large, and the shapability cannot be obtained.

As can be seen from the results of Comparative Example 3 and Comparative Examples 5 to 7, when the inorganic filler (C') which does not satisfy the requirements of the present invention is contained, the effect of reducing the fluidity is small, and appropriate shapability cannot be obtained. In addition, as can be seen from the results of Comparative Example 4, when a large amount of the inorganic filler (C') is blended, flowability and sag are suppressed and shapability is imparted, but the bending strength of the cured product is decreased, the contrast ratio increases, and the dental curable composition becomes opaque.

**Claims**

1. A dental curable composition comprising:

   a polymerizable monomer (A);
   an inorganic filler (B) having an average particle diameter of 50 nm to 1 $\mu$m, wherein a polarity of a zeta potential of the inorganic filler measured in water is either negative (minus) or positive (plus), and the polarity is constant between inorganic particles constituting the inorganic filler; and
   an inorganic filler (C) having a polarity of a zeta potential measured in water that is opposite to the polarity of the inorganic filler (B) and a specific surface area measured by a nitrogen adsorption method of 25 to 100 m$^2$/g, wherein blending amounts of the inorganic filler (B) and the inorganic filler (C) with respect to 100 parts by mass of the polymerizable monomer (A) are 170 to 270 parts by mass of the inorganic filler (B) and 5 to 50 parts by mass of the inorganic filler (C), respectively.

2. The dental curable composition according to claim 1, wherein a polarity of a zeta potential of the inorganic filler (B) measured in water is negative (minus).

3. The dental curable composition according to claim 1 or 2, wherein a proportion of a mass of the inorganic filler (C) to a total mass of the inorganic filler (B) and the inorganic filler (C) is 1 to 20% by mass.

4. The dental curable composition according to any one of claims 1 to 3, wherein the polymerizable monomer (A) is composed of a (meth)acrylate-based polymerizable monomer, the inorganic filler (B) is composed of silica-based composite oxide particles, and the inorganic filler (C) is composed of crystalline rare earth metal fluoride particles, wherein the particles have a full width at half maximum of a maximum intensity peak derived from the crystalline rare earth metal fluoride in an X-ray diffraction pattern of the particles of 0.3° or more.

5. A flowable composite resin comprising the dental curable composition according to any one of claims 1 to 4.

## INTERNATIONAL SEARCH REPORT

| International application No. |
| --- |
| **PCT/JP2022/041134** |

**A.    CLASSIFICATION OF SUBJECT MATTER**

  *A61K 6/887*(2020.01)i; *A61K 6/842*(2020.01)i; *A61K 6/878*(2020.01)i
  FI:    A61K6/887; A61K6/842; A61K6/878

According to International Patent Classification (IPC) or to both national classification and IPC

**B.    FIELDS SEARCHED**

Minimum documentation searched (classification system followed by classification symbols)

  A61K6/887; A61K6/842; A61K6/878

Documentation searched other than minimum documentation to the extent that such documents are included in the fields searched

  Published examined utility model applications of Japan 1922-1996
  Published unexamined utility model applications of Japan 1971-2023
  Registered utility model specifications of Japan 1996-2023
  Published registered utility model applications of Japan 1994-2023

Electronic data base consulted during the international search (name of data base and, where practicable, search terms used)

  JSTPlus/JMEDPlus/JST7580 (JDreamIII); CAplus/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
| --- | --- | --- |
| Y | WO 2019/131881 A1 (KURARAY NORITAKE DENTAL INC.) 04 July 2019 (2019-07-04) claims 1-13, paragraphs [0017], [0034], [0055], examples | 1-3, 5 |
| A | | 4 |
| Y | WO 2020/130143 A1 (KURARAY NORITAKE DENTAL INC.) 25 June 2020 (2020-06-25) paragraph [0142] | 1-3, 5 |
| A | JP 2014-177443 A (TOKUYAMA DENTAL CORP.) 25 September 2014 (2014-09-25) paragraph [0018], examples | 1-5 |
| A | JP 2020-11917 A (TOKUYAMA DENTAL CORP.) 23 January 2020 (2020-01-23) example 17 | 1-5 |

☐ Further documents are listed in the continuation of Box C.    ☑ See patent family annex.

| *    Special categories of cited documents: | "T"    later document published after the international filing date or priority date and not in conflict with the application but cited to understand the principle or theory underlying the invention |
| --- | --- |
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
| --- | --- |
| **04 January 2023** | **17 January 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2022/041134**

| Patent document cited in search report | | | Publication date (day/month/year) | Patent family member(s) | Publication date (day/month/year) |
|---|---|---|---|---|---|
| WO | 2019/131881 | A1 | 04 July 2019 | (Family: none) | |
| WO | 2020/130143 | A1 | 25 June 2020 | US 2022/0071852 A1 paragraph [0239] EP 3900694 A1 | |
| JP | 2014-177443 | A | 25 September 2014 | (Family: none) | |
| JP | 2020-11917 | A | 23 January 2020 | (Family: none) | |

Form PCT/ISA/210 (patent family annex) (January 2015)

**EP 4 434 512 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014083842 A
- WO 2020031444 A